(19) Europäisches Patentamt
European Patent Office
Office européen des brevets

(11) **EP 4 223 270 A1**

(12) **EUROPEAN PATENT APPLICATION**
published in accordance with Art. 153(4) EPC

(43) Date of publication:
**09.08.2023 Bulletin 2023/32**

(21) Application number: **21875890.2**

(22) Date of filing: **01.10.2021**

(51) International Patent Classification (IPC):
$A61K\ 6/78$ (2020.01)  $A61C\ 13/00$ (2006.01)
$A61C\ 13/083$ (2006.01)  $A61K\ 6/818$ (2020.01)

(52) Cooperative Patent Classification (CPC):
A61C 13/00; A61C 13/083; A61K 6/78; A61K 6/818

(86) International application number:
**PCT/JP2021/036503**

(87) International publication number:
**WO 2022/071594 (07.04.2022 Gazette 2022/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.10.2020 JP 2020168074**

(71) Applicant: **Kuraray Noritake Dental Inc.**
**Kurashiki-shi, Okayama 710-0801 (JP)**

(72) Inventors:
• **SAKAMOTO, Hiroyuki**
**Miyoshi-shi, Aichi 470-0293 (JP)**
• **KASHIKI, Nobusuke**
**Miyoshi-shi, Aichi 470-0293 (JP)**
• **KATO, Shinichiro**
**Miyoshi-shi, Aichi 470-0293 (JP)**

(74) Representative: **D Young & Co LLP**
**120 Holborn**
**London EC1N 2DY (GB)**

(54) **DENTAL CERAMIC COLORING SOLUTION**

(57)    The present invention provides a dental ceramic coloring solution with which shades with the desired yellow tint can be imparted to a dental ceramic without causing fading, even when the dental ceramic colored with the coloring solution is baked after building up porcelain.

The present invention relates to a coloring solution for coloring a dental ceramic, comprising a coloring component and a solvent, wherein the coloring component comprises a V component.

Processed by Luminess, 75001 PARIS (FR)

**Description**

TECHNICAL FIELD

The present invention relates to a coloring solution for dental ceramics. Specifically, the invention relates to a dental ceramic coloring solution suited for use in the fabrication of dental prostheses machined with a dental CAD/CAM system, for example, such as inlays, onlays, veneers, crowns, bridges, abutment teeth, dental posts, dentures, denture bases, and implant parts (fixtures, abutments).

BACKGROUND ART

Traditionally, metal has been used for a range of dental products, including, for example, dental prostheses (such as veneer crowns, dental caps, crowns, and post crowns), orthodontic products, and products for dental implants. However, metals lack aesthetic quality because of the colors that are distinctively different from the color of natural teeth, and can cause allergic reaction when released from these products. These issues involving the use of metal have been addressed by dental products that use ceramic materials such as aluminum oxide (alumina) and zirconium oxide (zirconia) as alternative materials of metal. Particularly, zirconia excels in strength, and has relatively good aesthetics, and this, combined in particular with the currently declining price of zirconia, has created a high demand for this material.

In recent years, there has been increasing use of a CAD/CAM system that makes use of a computer for designing and a milling machine for machining to form the final shape of a dental prosthesis, or to fabricate a prosthesis for large implants. For aesthetic values, the CAD/CAM system typically uses zirconia as a material of a mill blank to be milled. Particularly, recent years have seen increased use of zirconia that satisfies aesthetic requirements by reproducing the color of natural teeth with different shades of color vertically arranged in a thickness direction. For shades that are difficult to reproduce, a ceramic that has been worked into a shape of a dental prosthesis is colored by coating dental porcelain over a ceramic surface to satisfy high aesthetic requirements.

However, color coating of dental porcelain requires expertise and great technique. Specifically, high skills are required for coloring with dental porcelain because dental porcelain needs to be built up to develop colors with high aesthetics while reproducing the shape and structure of natural teeth with precision.

In a common technique used to circumvent the issues involved in the use of dental porcelain, a coloring solution is applied to a dental ceramic to achieve even better aesthetics and provide the look of natural teeth, instead of the skillful coloring with dental porcelain, or before using dental porcelain.

Normally, natural teeth have a yellow tint and a red tint. Techniques are proposed that give a yellow tint to ceramics, as follows.

For example, Patent Literature 1 discloses a Pr component and a Tb component as yellow-tinged coloring components for metal oxide ceramics. A coloring solution containing these coloring components was found to be capable of coloring zirconia itself. However, it was found that, when porcelain is built up and baked after coloring, zirconia colored by using this technique shows a pale yellow tint (fading) by the effects of the yellowing preventing agent (e.g., an antimony compound or a cerium compound) added into the porcelain.

Patent Literature 2 discloses a composite oxide of zirconium and vanadium as a colorant. However, while this composite oxide enables coloring in mixture form with a powder, the composite oxide is not applicable to coloring solutions because it is insoluble in solvents available for coloring solutions, and cannot evenly color zirconia when used as a coloring component of a coloring solution.

CITATION LIST

Patent Literature

Patent Literature 1: JP 2007-314536 A
Patent Literature 2: WO2016/068288

SUMMARY OF INVENTION

Technical Problem

As discussed above, Patent Literature 1 does not take into consideration the color failure that occurs when porcelain is built up, and involves ceramic fading. That is, improvements are needed if porcelain were to be used for build-up.

In Patent Literature 2, a composite oxide-containing colorant is contained in the substrate zirconia itself to give color. By using chromogenic zirconia as a raw material of a molded body, Patent Literature 2 solves the issue of fading- a type color failure that occurs when porcelain containing a yellowing preventing agent is built up on a zirconia sintered body-without using a coloring solution. That is, Patent Literature 2 is concerned with the relationship between substrate zirconia and porcelain containing a yellowing preventing agent. Accordingly, Patent Literature 2 assumes that substrate zirconia is containing a predetermined colorant, and does not indicate coloring with a coloring solution, aside from building up porcelain. Because the zirconia sintered body itself has the shade of natural teeth (paragraph 0116 in Patent Literature 2), any additional coloring procedures other than glazing and adding glossiness with porcelain would complicate the production process, and, considering industrial productivity, Patent Literature 2 does not probably assume any additional coloring.

When substrate zirconia contains a predetermined colorant as in Patent Literature 2, the zirconia sintered body itself has the shade of natural teeth, and baking of porcelain produces only small color changes. That is, in Patent Literature 2, the color of zirconia substrate is adjusted with the colorant contained within the substrate, and the zirconia sintered body has a predetermined color. Accordingly, another zirconia sintered body needs to be newly produced from a zirconia powder when the color needs to be changed as per patient's request. This led to investigations of ways to conveniently give color with a coloring solution when a substrate (for example, a zirconia pre-sintered body) to be milled into a dental prosthesis contains essentially no colorant, taking also into consideration the possibility of enabling easy fabrication of another dental prosthesis when giving a new color.

Nevertheless, coloring of a dental ceramic with a coloring solution still involves the fading issue due to the effects of the yellowing preventing agent (e.g., an antimony compound or a cerium compound) contained in the porcelain.

It is accordingly an object of the present invention to provide a coloring solution with which shades with the desired yellow tint can be imparted to a dental ceramic without causing fading, even when the dental ceramic colored with the coloring solution is baked after building up porcelain that contains a yellowing preventing agent containing antimony oxide and/or cerium oxide. Another object of the present invention is to provide a coloring solution that excels in ease of handling, and with which shades with the desired yellow tint can be imparted to a dental ceramic in a convenient fashion.

Solution to Problem

The present inventors conducted intensive studies to find a solution to the foregoing issues, and found that these issues can be solved with a coloring solution containing a V component. The present invention was completed after further studies.

Specifically, the present invention includes the following.

[1] A coloring solution for coloring a dental ceramic, comprising a coloring component and a solvent, wherein the coloring component comprises a V component.

[2] The coloring solution according to [1], wherein the V component is an ion or a complex.

[3] The coloring solution according to [1] or [2], wherein the V component has a valency of +IV and/or +V.

[4] The coloring solution according to any one of [1] to [3], wherein the V component is an oxidovanadium compound.

[5] The coloring solution according to any one of [1] to [4], wherein the V component is a component derived from at least one selected from the group consisting of vanadyl oxalate, vanadyl nitrate, and vanadyl acetate.

[6] The coloring solution according to any one of [1] to [5], wherein the content of the V component is 0.0100 to 45.0 mmol/L in terms of a V ion.

[7] The coloring solution according to any one of [1] to [6], wherein a dental ceramic after coloring and sintering has an a* of -20 to 20, and a b* of 5 to 60 in (L*,a*,b*) according to L*a*b*color system.

[8] The coloring solution according to any one of [1] to [7], wherein L* in (L*,a*,b*) according to L*a*b*color system is 60 to 95.

[9] The coloring solution according to any one of [1] to [8], wherein the solvent comprises water and/or an organic solvent.

[10] The coloring solution according to [9], wherein the organic solvent comprises at least one selected from the group consisting of an alcohol, a glycol, a triol, and a ketone.

[11] The coloring solution according to any one of [1] to [10], wherein the coloring component comprises a Ni component.

[12] The coloring solution according to any one of [1] to [11], wherein the coloring component comprises a Cr component.

[13] The coloring solution according to any one of [1] to [12], wherein the coloring solution further comprises a Zr component.

[14] The coloring solution according to any one of [1] to [13], wherein the coloring solution does not comprise a Pr component or a Tb component.

[15] The coloring solution according to any one of [1] to [14], wherein the dental ceramic comprises zirconia as a main component.

[16] A dental ceramic supporting a V component on a surface of the dental ceramic.

[17] The dental ceramic according to [16], wherein the V component is an ion or a complex.

[18] The dental ceramic according to [16] or [17], which comprises zirconia as a main component.

Advantageous Effects of Invention

With a dental ceramic coloring solution of the present invention, shades with the desired yellow tint can be imparted to a dental ceramic without causing fading, even when the dental ceramic colored with the coloring solution is baked after building up porcelain that contains a yellowing preventing agent containing antimony oxide and/or cerium oxide. The present invention can also provide a coloring solution that excels in ease of handling, and with which shades with the desired yellow tint can be imparted to a dental ceramic in a convenient fashion. A dental ceramic coloring solution of the present invention can reduce decrease of strength in a dental ceramic. The present invention also enables a dental prosthesis to have a different shade in a convenient fashion by allowing zirconia that does not contain a predetermined colorant to be conveniently milled with a CAD/CAM system using data for a dental prosthesis having the same substrate shape, when a need has arisen to fabricate another dental prosthesis with a new shade.

DESCRIPTION OF EMBODIMENTS

The present invention is a coloring solution for coloring a dental ceramic, comprising a coloring component and a solvent, wherein the coloring component comprises a V component.

V Component

The V (vanadium) component contained in a coloring solution of the present invention is described first. The V component comprises a V ion or a V complex, and is contained to color a dental ceramic in a yellow-tinged shade.

By applying a dental ceramic coloring solution containing a V component, shades with the desired yellow tint can be imparted to a dental ceramic without causing fading, even when the dental ceramic colored with the coloring solution is baked after building up porcelain that contains a yellowing preventing agent containing antimony oxide and/or cerium oxide. Though the reason for this remains unclear, the present inventors have proposed the following explanation. To describe specifically, a common composite oxide pigment does not easily form a solid solution with a dental ceramic because of its good stability, and it may not be possible to provide satisfactory colors or stable shades. The V component of the present invention, however, has a form of ions or complexes, and can provide a strong, stable color even when added in small amounts. Accordingly, the influence of the other components is small, and fading can be reduced.

Ions or complexes of V may be added in the coloring solution in the form of salts containing cations and anions of V, or in the form of complexes containing V and ligands. Examples of the anions or ligands include $OAc^-$, $NO_3^-$, $NO_2^-$, $CO_3^{2-}$, $HCO_3^-$, $ONC^-$, $SCN^-$, $SO_4^{2-}$, $SO_3^{2-}$, glutarate, lactate, gluconate, propionate, butyrate, glucuronate, benzoate, phenolate, halide anions (fluorides, chlorides, bromides), and acetate. Ac means acetyl group.

In view of stability and ease of handling, the vanadium compound is preferably a vanadium compound with a valency of +IV and/or +V, more preferably an oxidovanadium compound.

Specific examples of compounds added to contain the foregoing ions or complexes in a coloring solution of the present invention include:

vanadium compounds with a valency of III, such as vanadium(III) chloride anhydrous, vanadium(III) chloride hydrate (hexahydrate), vanadium(III) oxide, and vanadium(III) bromide; and

vanadium compounds with a valency of +IV or +V, such as vanadium acetylacetonate, vanadyl acetylacetonate, vanadyl stearate, vanadium naphthenate, vanadium benzoylacetonate, vanadyl oxalate, bis(maltolato)oxovanadium(IV), oxobis(1-phenyl-1,3-butanedionate)vanadium, vanadium(V) oxytriisopropoxide, vanadium(V) trichloride oxide, vanadium(IV) dichloride oxide, vanadium silicide, vanadium(IV) oxide, vanadium(V) oxide, iron tetrapolyvanadate, vanadyl(IV) sulfate hydrate, vanadyl oxalate (oxalic acid oxovanadium(IV) salt), vanadyl(IV) acetate ($VO[OC(O)CH_3]_2$), vanadyl(V) nitrate ($VO(NO_3)_3$), vanadyl glycolate, vanadium selenide, vanadium carbide (VC), vanadium nitride (VN), potassium divanadate, potassium vanadate, potassium metavanadate ($KVO_3$), sodium metavanadate ($NaVO_3$), sodium divanadate ($Na_4V_2O_7$), sodium vanadate ($Na_3VO_4$), sodium vanadate hydrate, lithium metavanadate ($LiVO_3$), rubidium divanadate ($Rb_4V_2O_7$), rubidium metavanadate ($RbVO_3$), rubidium vanadate ($Rb_3VO_4$), vanadium diborate, vanadium borate (VB), and vanadium(III) sulfide ($V_2S_3$).

In view of the ability to reduce fading, and good stability of the coloring solution and good ease of handling, preferred are vanadyl oxalate, vanadyl nitrate, and vanadyl acetate. The vanadium component may be used alone, or two or more thereof may be used in combination.

In view of developing a yellow tint, the content of the V component in the coloring solution is preferably 0.0100 to 45.0 mmol/L, more preferably 0.0200 to 40.0 mmol/L in the whole solution in terms of a V ion. In view of good stability of the V component, the content of the V component is even more preferably 0.0500 to 35.0 mmol/L, depending on the type of V component. In view of good stability of the V component, the content of the V component in certain preferred embodiments may be 0.0100 to 15.0 mmol/L, or 0.0200 to 10.0 mmol/L in the whole solution. When the content of the V component is 0.0100 mmol/L or more, it is possible to develop a sufficient yellow tint, and satisfy high aesthetic requirements. When the content of the V component is 45.0 mmol/L or less, it is possible to reproduce the color of natural teeth by preventing the development of an overly strong yellow tint. The content of the V component can be measured using a technique, for example, such as inductively coupled plasma (ICP) emission spectral analysis, or X-ray fluorescence analysis. In the present specification, the contents of metallic components can be measured by using these methods.

A coloring solution of the present invention may comprise other coloring components, provided that it does not hinder the effects of the present invention. Examples of such other coloring components include metal ion solutions.

The metal ion solutions contain colorative cations. The colorative cations are preferably ions of at least one element selected from the group consisting of Al, K, Cr, Na, Y, Gd, La, Yb, Tm, Ni, Mn, Co, Nd, Cu, and Er, more preferably ions of at least one element selected from the group consisting of Al, K, Cr, Na, La, Ni, Mn, Co, and Er, even more preferably ions of at least one element selected from the group consisting of Al, K, Cr, Na, Ni, Mn, Co, and Er. The metal ion solutions may contain one of these cations alone, or may contain two or more cations in combination.

The colorative cations may be contained in a solvent (described later) in the form of salts containing the above cations and anions. Examples of the anions include $OAc^-$, $NO_3^-$, $NO_2^-$, $CO_3^{2-}$, $HCO_3^-$, $ONC^-$, $SCN^-$, $SO_4^{2-}$, $SO_3^{2-}$, glutarate, lactate, gluconate, propionate, butyrate, glucuronate, benzoate, phenolate, halide anions (fluorides, chloride, bromide), and acetate.

A certain preferred embodiment is, for example, a coloring solution in which the coloring component comprises a Ni component. The Ni component can reproduce a reddish brown shade, and can provide the desired standard dental shade by combining yellow to dull brown. For example, the L* value in (L*,a*,b*) of L*a*b*color system may decrease when a coloring solution containing a Ni component is used. In other instances, the a* value may increase when a coloring solution containing a Ni component is used. In other cases, the b* value may decrease when a coloring solution containing a Ni component is used. The nickel compound may be a bivalent, trivalent, or tetravalent compound. Preferred are divalent nickel compounds. Examples of the Ni component include nickel(II) hydroxide, nickel(II) chloride hydrate (hexahydrate), nickel(II) nitrate hydrate (hexahydrate), nickel(II) sulfate hydrate (hexahydrate), and nickel(II) acetate hydrate (tetrahydrate). The Ni component may be used alone, or two or more thereof may be used in combination.

Another preferred embodiment is, for example, a coloring solution in which the coloring component contains a Cr component. The Cr component can reproduce a greenish gray shade, and can provide the desired standard dental shade by combining yellow to dull green. The chrome compound may be a trivalent or tetravalent compound. Preferred are trivalent chrome compounds. For example, the L* value in (L*,a*,b*) of L*a*b*color system may decrease when a coloring solution containing a Cr component is used. In other instances, the a* value may increase when a coloring solution containing a Cr component is used. In other cases, the b* value may decrease when a coloring solution containing a Cr component is used. Examples of the Cr component include chromium(III) chloride, chromium(III) chloride hydrate (hexahydrate), chromium(III) nitrate hydrate (nonahydrate), chromium(III) sulfate (n-hydrate), and chromium(III) acetate hydrate (monohydrate). In view of good solubility in water and in organic solvent, preferred are chromium(III) nitrate hydrate (nonahydrate), chromium(III) chloride hydrate (hexahydrate), and chromium(III) acetate hydrate (monohydrate). The Cr component may be used alone, or two or more thereof may be used in combination.

A coloring solution of the present invention may comprise other metallic components, provided that it does not hinder the effects of the present invention. For example, the strength of ceramic after the application of the coloring solution may improve when the coloring solution comprises a Zr component. Another embodiment is, for example, a coloring solution that does not comprise a Pr component or a Tb component.

The Zr component comprises a Zr ion or a Zr complex. Ions or complexes of Zr may be added in the coloring solution in the form of salts containing cations and anions of Zr, or in the form of complexes containing Zr and its ligands. Examples of the anions include $OAc^-$, $NO_3^-$, $NO_2^-$, $CO_3^{2-}$, $HCO_3^-$, $ONC^-$, $SCN^-$, $SO_4^{2-}$, $SO_3^{2-}$, glutarate, lactate, gluconate, propionate, butyrate, glucuronate, benzoate, phenolate, halide anions (fluorides, chloride, bromide), and acetate.

Specific examples of compounds added to contain the foregoing ions or complexes in a coloring solution of the present invention include zirconium(IV) chloride oxide hydrate, zirconium(IV) sulfate, zirconium(IV) tetrakis(acetylacetonate), zirconium(IV) chloride, zirconium(IV) octanoate, zirconium(IV) oleate oxide, dichlorobis(η5-cyclopentadienyl)zirconium(IV), zirconium(IV) acetate oxide, zirconium(IV) oxide hydrate, zirconium(IV) stearate oxide, zirconium(IV) nitrate oxide hydrate, zirconium(IV) n-butoxide, zirconium(II) hydroxide, zirconium(IV) carbide, dizirconium carbonate trioxide hydrate, zirconium nitride, ammonium hexafluorozirconate(IV), zirconium(IV) iodide, zirconium(IV) laurate oxide ($Zr(C_{11}H_{23}COO)_2O$), zirconium(IV) sulfate hydrate, and zirconium(IV) dihydrogen phosphate oxide. Preferred among these compounds are zirconium(IV) chloride oxide hydrate, zirconium(IV) chloride, zirconium(IV) acetate oxide, zirconium(IV) oxide hydrate, and zirconium(IV) nitrate oxide hydrate, more preferably zirconium(IV) chloride oxide hydrate. The Zr component may be used alone, or two or more thereof may be used in combination.

In view of improving the strength of a dental ceramic, the content of the Zr component in the coloring solution is preferably 0.0650 to 0.900 mol/L, more preferably 0.0800 to 0.850 mol/L, even more preferably 0.0900 to 0.800 mol/L in the whole solution in terms of a Zr ion. When the content of the Zr component is 0.0650 mol/L or more, it is possible to provide the effect to improve the strength of a dental ceramic. When the content of the Zr component is 0.900 mol/L or less, the strength can improve without decreasing the aesthetics of the dental ceramic.

Preferably, a coloring solution of the present invention comprises water and/or an organic solvent as solvent. Water and/or an organic solvent allow the coloring component and the Zr component to dissolve, and improve the penetration of the coloring solution into a dental ceramic. The solvent content in the coloring solution is preferably 45.0 to 99.9999 mass%, more preferably 60 to 99.9995 mass%, even more preferably 75 to 99.9990 mass%.

It is required that water be essentially free of impurities that are detrimental to the effects of the present invention. Preferably, water is purified water, distilled water, ion-exchange water, or deionized water. The content of water in the coloring solution is preferably 45.0 to 99.9999 mass%, more preferably 60 to 99.9995 mass%, even more preferably 75 to 99.9990 mass%.

The organic solvent may be any solvent that can dissolve the cations, and is preferably an alcohol, a glycol, a triol, or a ketone, or a mixture selected from combinations of these organic solvents. Specific examples include:

alcohols such as methanol, ethanol, 1-propanol, 2-propanol, isopropanol, 1-butanol, 2-butanol, 1-heptanol, 2-heptanol, 3-heptanol, 1-hexanol, 2-hexanol, 3-hexanol, 2-methyl-1-pentanol, 3-methyl-1-pentanol, 4-methyl-1-pentanol, 2-methyl-2-pentanol, 2-methyl-3-pentanol, 2,2-dimethyl-1-butanol, 2-ethyl-1-butanol, ethylene glycol monomethyl ether, ethylene glycol monoethyl ether, ethylene glycol monobenzyl ether, propylene glycol monomethyl ether, propylene glycol monoethyl ether, ethylene glycol monobutyl ether, diethylene glycol monomethyl ether, diethylene glycol monoethyl ether, diethylene glycol monobenzyl ether, propylene glycol monopropyl ether, tripropylene glycol monomethyl ether, diethylene glycol monobutyl ether, triethylene glycol monomethyl ether, benzyl alcohol, 2-(benzyloxy)ethanol, 3-(benzyloxy)-1-propanol, 2-(benzyloxy)-1-butanol, and 5-(benzyloxy)-1-pentanol;

diols such as 1,2-ethanediol, 1,2-propanediol, 1,3-propanediol, 1,2-butanediol, 1,3-butanediol, 1,4-butanediol, 2,3-butanediol, 1,2-pentanediol, 1,5-pentanediol, 2,4-pentanediol, 1,2-hexanediol, 2,5-hexanediol, ethylene glycol, diethylene glycol, triethylene glycol, tetraethylene glycol, polyethylene glycol (a molecular weight of 200 to 600), propylene glycol, dipropylene glycol, polypropylene glycol, 1-methyl-1,3-propanediol, 2-methyl-1,3-propanediol, 2-methyl-1,4-butanediol, 3-methyl-1,3-butanediol, 2-methyl-2,4-pentanediol, 3-methyl-1,5-pentanediol, 2,4-diethyl-1,5-pentanediol, and 2-ethyl-1,3-hexanediol;

triols such as glycerin, 1,2,4-butanetriol, 1,2,3-butanetriol, and 1,2,6-hexanetriol; and

ketones such as acetone, 2-butanone, 2-pentanone, isobutyl ketone, diisobutyl ketone, and cyclohexanone.

These organic solvents may be used alone, or two or more thereof may be used in an appropriate combination. The organic solvent may be used as a thickener (described later) to adjust viscosity.

The solvent content in the coloring solution is preferably 45.0 to 99.9999 mass%, more preferably 60.0 to 99.9995 mass%, even more preferably 75.0 to 99.9990 mass%.

The coloring solution may comprise a complexing agent to such an extent that it does not hinder the effects of the present invention. Adding a complexing agent may be beneficial in improving the storage stability of the coloring solution, accelerating the dissolution process of the salts added to the coloring solution, and/or increasing the amount of salts that can dissolve in the coloring solution.

The complexing agent can typically form complexes with the metal ions present in the coloring solution. The complexes formed must be soluble in solvent. For example, the complexing agent may be used in at least a stoichiometric proportion in relation to the molar quantity of the ions contained in the coloring solution. Desirable results can be obtained when the mole ratio of the complexing agent is about 1 or about 2, or about 3 or more relative to the cations in the coloring solution.

Examples of the complexing agent include N,N-di(2-hydroxyethyl)glycine, acetylacetonate, crown ether, cryptands, ethylenediaminetriacetate and salts thereof, ethylenediaminetetraacetate and salts thereof, nitrilotriacetate and salts thereof, citric acid and salts thereof, triethylenetetraamine, porphin, polyacrylate, polyaspartate, acidic peptides, phthalocyanine, salicylate, glycinate, lactate, propylenediamine, ascorbate, oxalic acid and salts thereof, and a mixture of these. The complexing agent may be used alone, or two or more thereof may be used in an appropriate combination.

The content of the complexing agent in the coloring solution is not particularly limited, as long as the present invention can exhibit its effects. For example, it is preferable to contain the complexing agent in an amount sufficient to dissolve the cations in the solution, or to prevent precipitation of the cations. Specifically, the content of the complexing agent in the coloring solution is preferably 0.01 mass% or more, more preferably 0.05 mass% or more, even more preferably 0.10 mass% or more. There is no specific upper limit for the content of the complexing agent. However, the content of the complexing agent is preferably 50 mass% or less, more preferably 20 mass% or less, even more preferably 10 mass% or less. The complexing agent may fail to fully dissolve the cations when used in excessively small amounts, whereas the excess portion of the complexing agent itself may remain without dissolving when the complexing agent is used in excessively large amounts.

A coloring solution of the present invention has a pH of preferably 0 to 9, more preferably 1 to 7, even more preferably 2 to 6. The cations may start precipitating from the solution when the pH falls outside of these ranges. For example, the pH is preferably 0 to 9 when the coloring solution is an aqueous solution. The pH is preferably 0 to 6 when the coloring solution does not contain a complexing agent, and is preferably 3 to 9 when the coloring solution contains a complexing agent. The pH can be measured with a commercially available pH meter (for example, a compact pH meter LAQUAtwin manufactured by Horiba Ltd.).

Preferably, a coloring solution of the present invention has an appropriate viscosity so that the solution can be applied to a ceramic surface in necessary amounts, or can move into the pores of a ceramic unfired body (also referred to as "molded body" in the present specification) or a ceramic pre-sintered body. The appropriate viscosity is, for example, preferably 0.1 to 10,000 mPa, more preferably 0.5 to 6,000 mPa, even more preferably 1 to 3,000 mPa at 20°C. When the viscosity is too high, it may not be possible to contain the coloring solution in the pores of a ceramic unfired body or a ceramic pre-sintered body. The viscosity can be measured at 25°C with a Brookfield viscometer, though the method of viscosity measurement is not particularly limited.

In order to achieve the appropriate viscosity, a coloring solution of the present invention may comprise a thickener to such an extent that it does not hinder the effects of the present invention.

The thickener may be selected from the above organic solvents to adjust viscosity, or may be selected from the following thickeners. Examples of thickeners other than the organic solvents include:

polysaccharide compounds such as methyl cellulose, carboxycellulose, hydroxyethyl cellulose, xanthan gum, guar gum, carrageenan, tamarind seed gum, and pectin;
sugar alcohol compounds such as sorbitol, erythritol, xylitol, and trehalose;
synthetic polyol compounds such as diglycerin, triglycerin, polyglycerin, and polyvinyl alcohol; and
solid organic compounds such as sodium polyacrylate, ammonium polyacrylate, polyethylene oxide, polyethylene glycol (a molecular weight of 1,000 or more), polyvinylpyrrolidone, calcium stearate, magnesium stearate, zinc stearate, aluminum stearate, polyethylene glycol monostearate, 12-hydroxystearic acid, stearamide, oleamide, and ethylenebisoleamide. These thickeners may be used alone, or two or more thereof may be used in an appropriate combination.

The content of the thickener in a coloring solution of the present invention is preferably 0.01 to 10 mass%, more preferably 0.02 to 8 mass%, even more preferably 0.05 to 5 mass%.

A coloring solution of the present invention may comprise other additives, provided that it does not hinder the effects of the present invention.

Examples of the additives include stabilizers (for example, methoxyphenol, hydroquinone, Topanol A (2,4-dimethyl-6-tert-butylphenol), and a mixture of these (excluding a stabilizer capable of reducing a phase transformation of zirconia)), buffers (for example, acetate, amino buffer, and a mixture of these), preservatives (for example, sorbic acid, benzoic acid, and a mixture of these), and a mixture of these. The additives may be used alone, or two or more thereof may be used in combination.

The content of the additive in a coloring solution of the present invention may be, for example, 0.01 to 10 mass%, 0.05 to 5 mass%, or 0.1 to 3 mass%.

In a coloring solution of the present invention, the coloring component may comprise a colorant that becomes decolored after zirconia is fired. The colorant that becomes decolored after zirconia is fired is not particularly limited, as long as it is decolored after firing zirconia, and can provide a satisfactory color difference before and after firing. The colorant may be, for example, an organic dye.

The organic dye is not particularly limited, as long as it is an organic dye having a chromophore, and that dissolves in the coloring solution. Preferably, the organic dye is an aromatic organic dye, that is, an organic dye containing one or more aromatic groups that may be substituted. More preferably, the organic dye is an aromatic organic dye having an auxochrome, in addition to a chromophore. The chromophore is not particularly limited, as long as it is a group of atoms that confer color by binding to an aromatic ring. Examples include a nitro group, an azo group, a ketimide group ($>C=N-$), a carbonyl group, a carbon-carbon double bond, a carbon-carbon triple bond, a carbon-nitrogen multiple bond, a thiocarbonyl group, a nitroso group, and an azoxy group. The organic dye may contain one of such groups of atoms alone, or may contain two or more groups of atoms in an appropriate combination. Examples of the auxochrome include a hydroxyl group, an amino group, a carboxyl group, a sulfone group, and a halogen atom. The organic dye may contain one of such auxochromes alone, or may contain two or more auxochromes in an appropriate combination.

The organic dye is preferably a food dye, more preferably a food dye that dissolves in the coloring solution, because the colorant that becomes decolored after firing zirconia must be harmless and nontoxic to humans. Examples of such food dyes include:

organic dyes containing two or more aromatic groups, such as yellow 4 (tartrazine), yellow 5 (sunset yellow FCF), red 2 (amaranth), red 102 (new coccin), blue 1 (brilliant blue FCF), blue 2 (indigo carmine), green 3 (fast green FCF), and red 102 (new coccin);

organic dyes containing fused aromatic groups with a xanthene scaffold (xanthene dye), such as acid red 289, bromopyrogallol red, rhodamine B, rhodamine 6G, rhodamine 6GP, rhodamine 3GO, rhodamine 123, eosin (eosin B, eosin Y), fluorescein, and fluorescein isothiocyanate;

cochineal dyes (carmic acid dyes); and

betalain dyes such as beet red (main components: isobetanin and betanin), betanin, isobetanin, probetanin, and neobetanin.

Preferred are organic dyes containing two or more aromatic groups and having a ketimide group or an azo group as a chromophore, for example, such as yellow 4 (tartrazine), yellow 5 (sunset yellow FCF), red 2 (amaranth), red 102 (new coccin), blue 1 (brilliant blue FCF), green 3 (fast green FCF), and isobetanin. Because the colorant (A) can be changed depending on the content of the stabilizer in a zirconia pre-sintered body to which a coloring solution of the present invention is applied, a certain preferred embodiment is, for example, a zirconia coloring solution in which the colorant (A) is an organic dye containing two or more aromatic groups, and having a ketimide group or an azo group as a chromophore, and a sulfone group as an auxochrome. In the present specification, "aromatic group" includes an aromatic group whose ring structure consists solely of carbon atoms, and a heteroaromatic group whose ring structure contains elements other than carbon (e.g., oxygen, nitrogen). The colorant that becomes decolored after firing zirconia may be used alone, or two or more thereof may be used in an appropriate combination. The colorant that becomes decolored after firing zirconia may produce different color intensities depending on the pH of the coloring solution, and may take different compound structures for different pH values. However, the pH of the zirconia coloring solution is not particularly limited, as long as the present invention can exhibit its effects. A PH value may be selected that provides an appropriate color intensity, according to the type of the colorant that becomes decolored after firing zirconia.

The content of the colorant that becomes decolored after firing zirconia is not particularly limited, as long as the liquid component can produce color. Preferably, the content of the colorant that becomes decolored after firing zirconia is 0.009 to 3.0 mass%, more preferably 0.09 to 1.6 mass%, even more preferably 0.2 to 1.4 mass%, particularly preferably 0.25 to 1.2 mass% relative to the mass of the whole coloring solution.

A certain embodiment is, for example, a coloring solution in which the coloring component is essentially free of a colorant that becomes decolored after firing zirconia. By "essentially free of a colorant that becomes decolored after firing zirconia", it means that the content of the colorant that becomes decolored after firing zirconia is preferably less than 0.009 mass%, more preferably less than 0.001 mass%, even more preferably less than 0.0001 mass% relative to the mass of the whole coloring solution. The content of the colorant that becomes decolored after firing zirconia may be 0 mass%.

With a coloring solution of the present invention, the yellow shade needed for dental use can be imparted to a dental ceramic. A dental ceramic after coloring and sintering has chromaticity (L*,a*,b*) with a* of preferably -20 to 20, more preferably -18 to 18, even more preferably -15 to 15 according to L*a*b*color system. Preferably, b* is 5 to 60, more preferably 10 to 58, even more preferably 12 to 55. Preferably, L* is 60 to 95, more preferably 68 to 92, even more preferably 65 to 90. The method of measurement of chromaticity (L*,a*,b*) is as described in the EXAMPLES section below.

A dental ceramic colored with a coloring solution of the present invention is not particularly limited, as long as it contains ceramic. Examples include those containing, for example, zirconia (zirconium oxide or $ZrO_2$ as it is also called), alumina (aluminum oxide or $Al_2O_3$ as it is also called), feldspar glass, disilicate glass, or porcelain. Preferred as dental ceramics are those containing zirconia and/or alumina, more preferably those containing zirconia as a main component. In the case of a dental ceramic containing zirconia as a main component, the zirconia content is more preferably 65 mass% or more, particularly preferably 75 mass% or more, most preferably 85 mass% or more.

A dental ceramic colored with a coloring solution of the present invention may be an unfired body or a pre-sintered body, provided that it is not sintered. However, in view of penetration of the coloring solution, a zirconia pre-sintered body is preferred in the case of a dental ceramic containing zirconia as a main component.

Another embodiment of the present invention is, for example, a dental ceramic (a colored ceramic pre-sintered body or unfired body) supporting a V component on a surface of the dental ceramic. The content of the V component is not particularly limited, as long as the present invention can exhibit its effects. For example, the content of the V component can be appropriately adjusted by adjusting the applied amount of a coloring solution of the present invention, according to, for example, the desired color intensity to be obtained after sintering. The V component can be adjustably supported in an appropriate fashion not only on the outermost surface but in the surface of a ceramic pre-sintered body or unfired body because the V component can penetrate into a ceramic pre-sintered body or unfired body by capillary action through spaces that are in communication with outside, following application of, for example, a coloring solution of the present invention. By "support", it generally means a state of adhesion to a support. In the present invention, "support" refers to a state of adhesion to a ceramic by means of, for example, adsorption.

With a dental ceramic of the present invention, the desired yellow-tinged shade can be imparted to the dental ceramic while reducing fading, even when the dental ceramic is baked after building up porcelain that contains a yellowing preventing agent containing antimony oxide and/or cerium oxide.

Preferably, the ceramic pre-sintered body or unfired body comprises zirconia as a main component, as noted above. Below is a description of zirconia. In the present invention, a pre-sintered body after coloring is called "colored zirconia pre-sintered body" to distinguish it from "zirconia pre-sintered body", a simplified term used herein to refer to a pre-sintered body before coloring with the coloring solution. A coloring solution of the present invention can also be used to color a zirconia unfired body. In this case, a zirconia sintered body is produced without producing a pre-sintered body. In situations assuming such sintered bodies, the conditions in the descriptions given below in conjunction with zirconia pre-sintered bodies are equally applicable as preferred embodiments of the zirconia unfired body.

The following describes a zirconia pre-sintered body of the present invention. The zirconia pre-sintered body refers to a material containing zirconia ($ZrO_2$: zirconium oxide) as a main component, and in which zirconia has pre-sintered after the material was molded according to the dental product to be produced. For example, "zirconia pre-sintered body" means a block formed while zirconia particles (powder) are not fully sintered. The main component may be 50 mass% or more. The zirconia content in a zirconia pre-sintered body according to the present invention is preferably 60 mass% or more, more preferably 70 mass% or more, even more preferably 80 mass% or more. For example, for applications as dental prostheses or dental implant products, the zirconia pre-sintered body can be prepared by, for example, pre-sintering a disc or a block obtained by press forming a zirconia powder using a known technique. The zirconia pre-sintered body has a density of preferably 2.7 g/cm$^3$ or more. The zirconia pre-sintered body has a density of preferably 4.0 g/cm$^3$ or less, more preferably 3.8 g/cm$^3$ or less, even more preferably 3.6 g/cm$^3$ or less. Molding can be performed with ease when the density is confined in these ranges. The density of the pre-sintered body can be calculated as, for example, a ratio of the mass of the pre-sintered body to the volume of the pre-sintered body. The zirconia pre-sintered body has a three-point flexural strength of preferably 15 to 70 MPa, more preferably 18 to 60 MPa, even more preferably 20 to 50 MPa. The flexural strength can be measured following ISO 6872:2015 except for the specimen size, using a specimen measuring 5 mm in thickness, 10 mm in width, and 50 mm in length. For surface finishing, the specimen surfaces, including chamfered surfaces (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 0.5 mm/min.

It is preferable that the zirconia in the zirconia pre-sintered body and in a zirconia powder and a molded body of zirconia powder be predominantly monoclinic in crystal system. In the present invention, "being predominantly monoclinic in crystal system" means that the fraction $f_m$ of the monoclinic crystal system of zirconia calculated from the formula (1) below is 50% or more relative to the total amount of all the crystal systems (monoclinic, tetragonal, and cubic) of zirconia. In the zirconia pre-sintered body and in a zirconia powder and a molded body of zirconia powder, the fraction $f_m$ of the monoclinic crystal system of zirconia calculated from the formula (1) below is preferably 55% or more, more preferably 60% or more, even more preferably 70% or more, yet more preferably 75% or more, particularly preferably 80% or more, still more preferably 85% or more, most preferably 90% or more relative to the total amount of the monoclinic, tetragonal, and cubic crystal systems. The fraction $f_m$ of monoclinic crystal system can be calculated from the formula (1) below, using peaks in an X-ray diffraction (XRD) pattern by CuKα radiation. The predominant crystal system possibly contributes to elevating the shrinkage temperature in firing the zirconia pre-sintered body, and reducing the firing time.

In the zirconia pre-sintered body, the peaks for tetragonal and cubic crystal systems may be essentially undetectable. That is, the fraction $f_m$ of the monoclinic crystal system may be 100%.

[Math. 1]

$$f_m(\%) = \frac{I_m(111) + I_m(11-1)}{I_m(111) + I_m(11-1) + I_t(111) + I_c(111)} \times 100 \tag{1}$$

In formula (1), $I_m(111)$ and $I_m(11-1)$ represent the peak intensities of the (111) plane and (11-1) plane, respectively, of the monoclinic crystal system of zirconia, $I_t(111)$ represents the peak intensity of the (111) plane of the tetragonal crystal system of zirconia, and $I_c(111)$ represents the peak intensity of the (111) plane of the cubic crystal system of zirconia.

Preferably, a zirconia pre-sintered body of the present invention comprises a stabilizer capable of reducing a phase transformation of zirconia. For example, a stabilizer capable of reducing a phase transformation of zirconia is preferably contained in zirconia before pre-sintering.

Examples of the stabilizer capable of reducing a phase transformation of zirconia include oxides such as yttrium oxide ($Y_2O_3$; hereinafter "yttria"), calcium oxide (CaO), magnesium oxide (MgO), yttria, cerium oxide ($CeO_2$), scandium oxide ($Sc_2O_3$), niobium oxide ($Nb_2O_5$), lanthanum oxide ($La_2O_3$), erbium oxide ($Er_2O_3$), samarium oxide ($Sm_2O_3$), europium oxide ($Eu_2O_3$), and thulium oxide ($Tm_2O_3$). Preferred is yttria. These may be used alone, or two or more thereof may be used in combination. In a certain preferred embodiment of the coloring solution, a dental ceramic to be colored contains zirconia as a main component, and yttria as a stabilizer, and the stabilizer consists essentially of yttria In such a preferred embodiment, "stabilizer consisting essentially of yttria" means that the content of stabilizers other than yttria is less than 0.1 mol%, preferably 0.05 mol% or less, more preferably 0.01 mol% or less, even more preferably 0.001 mol% or less in total 100 mol% of zirconia and stabilizer.

When the zirconia pre-sintered body contains a stabilizer, the stabilizer content is preferably 0.1 to 18 mol%, more preferably 1 to 15 mol%, even more preferably 1.5 to 10 mol% in total 100 mol% of zirconia and stabilizer. A certain preferred embodiment includes a dental ceramic and a coloring solution for coloring same, in which the dental ceramic contains zirconia as a main component, and the yttria content is 1.5 to 10 mol% relative to the total mole of zirconia and yttria. In such a preferred embodiment, the yttria content is preferably 2.0 to 9.0 mol%, more preferably 2.5 to 8.5 mol%, even more preferably 2.8 to 8.0 mol%.

The zirconia pre-sintered body may optionally comprise, for example, a colorant (including a pigment, a composite pigment, and a fluorescent agent), alumina ($Al_2O_3$), titanium oxide ($TiO_2$), or silica ($SiO_2$). These components may be used alone, or two or more thereof may be used as a mixture. Examples of the pigment include an oxide of at least one element selected from the group consisting of Ti, V, Cr, Mn, Fe, Co, Ni, Zn, Y, Zr, Sn, Sb, Bi, Ce, Sm, Eu, Gd, and Er. Examples of the composite pigment include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$. Examples of the fluorescent agent include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A typical method of production of the zirconia pre-sintered body is as follows. First, granules are prepared that comprise a zirconia raw material containing a stabilizer (preferably, zirconia particles that are predominantly monoclinic in crystal system), and the granules are pressed into a shape such as a block or a disc. Optionally, the molded body is subjected to CIP (Cold Isostatic Pressing). The applied pressure is, for example, 50 to 500 MPa. This is followed by pre-sintering. Pre-sintering can produce a zirconia pre-sintered body by gradually increasing temperature from room temperature to 800 to 1,200°C, and retaining this temperature for about 1 to 6 hours. The resulting zirconia pre-sintered body can then be milled with a known device, according to the final dental product. For example, when the dental product is a dental prosthesis, the zirconia pre-sintered body is milled into a shape of a dental cap with a CAD/CAM or other devices.

The zirconia pre-sintered body may be a commercially available product. Examples of such commercially available products include Noritake KATANA® zirconia (Model: Disc UTML, Disc STML, Disc ML, Disc HT, Disc LT; all manufactured by Kuraray Noritake Dental Inc.).

A method of production of a colored zirconia pre-sintered body of the present invention comprises the step of containing the coloring solution in a zirconia pre-sintered body after milling. The coloring solution may be contained by, for example, being applied to the zirconia pre-sintered body with a brush or the like, dipping the zirconia pre-sintered body in a container containing the coloring solution, or spraying the coloring solution to the zirconia pre-sintered body with a sprayer or like. Known tools and devices can be used. When a zirconia sintered body is produced directly from an unfired body without the pre-sintering step, the coloring solution may be contained in the zirconia unfired body after milling.

The present invention also encompasses a zirconia sintered body formed from the colored zirconia pre-sintered body. A method of production of the zirconia sintered body comprises the step of firing the colored zirconia pre-sintered body. The firing temperature (highest temperature in firing) can be appropriately selected according to the type of zirconia, and is not particularly limited as long as the Er component and Co component contained in the coloring solution can develop color. However, the firing temperature is preferably 1,350°C or more, more preferably 1,450°C or more, even more preferably 1,500°C or more. The upper limit of firing temperature is not particularly limited, and is, for example, preferably 1,700°C or less. A zirconia sintered body of the present invention encompasses not only sintered bodies after sintering of molded zirconia particles under ordinary pressure or no applied pressure, but sintered bodies compacted by a high-temperature pressing process such as HIP (Hot Isostatic Pressing).

The stabilizer content in the zirconia sintered body can be measured, for example, by inductively coupled plasma (ICP) emission spectral analysis or X-ray fluorescence analysis.

Preferably, the zirconia sintered body has at least one of partially stabilized zirconia and fully stabilized zirconia as the matrix phase. In the zirconia sintered body, the predominant crystalline phase of zirconia is at least one of the tetragonal crystal system and the cubic crystal system. The zirconia sintered body may have both the tetragonal crystal system and the cubic crystal system. Preferably, the zirconia sintered body is essentially free of a monoclinic crystal system. Zirconia that is partially stabilized by addition of a stabilizer is called partially stabilized zirconia (PSZ), whereas zirconia that is fully stabilized is called fully stabilized zirconia.

The present invention encompasses dental products formed from the zirconia sintered body. Examples of the dental products include dental prostheses, orthodontic products, and dental implant products. The dental prostheses can be used as, for example, zirconia inlays, onlays, laminate veneers, or crowns.

In all of the foregoing embodiments, aspects such as the type and content of each component can be changed as appropriate, and changes such as additions and deletions can be made in any of the components. In all of the foregoing embodiments, the composition and property values of the coloring solution can be varied and combined as appropriate.

The present invention encompasses embodiments combining the foregoing features, provided that the present invention can exhibit its effects with such combinations made in various forms within the technical scope of the present invention.

EXAMPLES

The following describes the present invention in greater detail by way of Examples. The present invention, however, is not limited by the following descriptions.

Examples 1 to 18 and Comparative Examples 1 and 2

Coloring solutions were prepared for Examples and Comparative Examples, and their properties were evaluated, as follows. The results are presented in Tables 1 and 2.

Preparation of Coloring Solution

The components shown in Tables 1 and 2 were mixed at ordinary temperature in the amounts shown in Tables 1 and 2 to prepare coloring solutions. The molar concentration of each metallic component in the coloring solutions was measured by inductively coupled plasma (ICP) emission spectral analysis (SPS3500, manufactured by Hitachi High-Technologies Corporation).

Evaluation of Ease of Handling of V Component

The V component was evaluated for its ease of handling and stability during the preparation of coloring solutions, using the following criteria.

Good: No visually observable changes such as white smoke or discoloration in the air at ordinary temperature; there is no problem in handling

Moderate: White smoke is visually observable in the air at ordinary temperature; some care must be taken in handling

Poor: Changes such as white smoke or discoloration, or marked heat generation are visually observable in the air at ordinary temperature; handling is difficult

Production of Zirconia Pre-Sintered Body

The following describes production of a zirconia pre-sintered body to which the coloring solution is applied.

First, a zirconia powder containing a stabilizer was prepared. A mixture was prepared by adding 9.9 mass% (5.5 mol%) of yttria as stabilizer to 90.1 mass% of a zirconia powder that is predominantly monoclinic in crystal system. The mixture was added to water to prepare a slurry, and the slurry was mixed and pulverized to an average particle diameter of 0.13 $\mu$m or less by wet pulverization with a ball mill. After pulverization, the slurry was dried with a spray dryer, and the resulting powder was fired at 950°C for 2 hours to prepare a powder (primary powder). The average particle diameter can be determined by a laser diffraction scattering method. As a specific example of a laser diffraction scattering method, a 0.2% aqueous solution of sodium hexametaphosphate may be used as a dispersion medium for the measurement of average particle diameter by volume, using a laser diffraction particle size distribution analyzer (SALD-2300, manufactured by Shimadzu Corporation).

Water was added to the primary powder to prepare a slurry, and the slurry was mixed and pulverized to an average particle diameter of 0.13 $\mu$m or less by wet pulverization with a ball mill. After adding a binder to the pulverized slurry, the slurry was dried with a spray dryer to prepare a powder (secondary powder). The secondary powder was used as a raw material powder for the production of the zirconia pre-sintered body below.

The method of production of a zirconia pre-sintered body is as follows. The raw material powder (1.32 g) was filled into a die measuring 19 mm in diameter, and was pressed under a surface pressure of 57.5 kN for 20 seconds with a uniaxial pressing machine (primary press forming). The resulting primary press-molded body was then fired at 1,000°C for 2 hours to prepare a zirconia pre-sintered body.

Chromaticity Measurement after Firing

The coloring solution prepared was applied to the zirconia pre-sintered body produced, and the zirconia pre-sintered body was fired into a sintered body under the firing conditions shown in Tables 1 and 2. The sintered body was ground into a circular disc measuring 15 mm in diameter and 1.2 mm in thickness, and was measured for chromaticity against a white background according to L*a*b*color system (JIS Z 8781-4:2013 Color Measurements - Part 4: CIE 1976 L*a*b* color space), using a spectrophotometer (Crystaleye CE100-DC/JP, 7-band LED light source) manufactured by Olympus Corporation under this trade name. The chromaticity was measured as L1*, a1*, and b1* (n = 3). Tables 1 and 2 show the mean values of measured values.

Measurement of Biaxial Flexural Strength after Firing

The coloring solution prepared was applied to the zirconia pre-sintered body produced, and the zirconia pre-sintered body was fired into a sintered body (15 mm in diameter, 1.2 mm in thickness) under the firing conditions shown in Tables 1 and 2. The sintered body was measured for biaxial flexural strength in compliance with JIS T 6526:2012 at a crosshead speed of 0.5 mm/min, using a desktop universal precision tester Autograph (AG-I 100kN) manufactured by Shimadzu Corporation under this trade name (n = 5). Tables 1 and 2 show the mean values of measured values.

Chromaticity Measurement after Porcelain Build-Up and Firing

The coloring solution prepared was applied to the zirconia pre-sintered body produced, and the zirconia pre-sintered body was fired under the firing conditions shown in Tables 1 and 2. After firing, Cerabien® ZR external stain E Glaze (manufactured by Kuraray Noritake Dental Inc.) was built up, and a sintered body was obtained by firing under the firing conditions shown in Tables 1 and 2. The sintered body was ground into a circular disc measuring 15 mm in diameter and 1.2 mm in thickness, and was measured for chromaticity against a white background according to L*a*b*color system (JIS Z 8781-4:2013 Color Measurements - Part 4: CIE 1976 L*a*b* color space), using a spectrophotometer (Crystaleye CE100-DC/JP, 7-band LED light source) manufactured by Olympus Corporation under this trade name. The chromaticity was measured as L2*, a2*, and b2* (n = 3). Tables 1 and 2 show the mean values of measured values.

Evaluation of Shade Difference Before and After Firing Porcelain

For evaluation of fading, the measured values of L1*, a1*, and b1* and the measured values of L2*, a2*, and b2* were substituted in the following formulae (2) and (3) to determine $\Delta b^*$, which is an index of yellow tint change, and $\Delta E^*$, which is an index of discoloration.

$$\Delta b^* = b2^* - b1^* \qquad (2)$$

$$\Delta E^* = \{(L2^* - L1^*)^2 + (a2^* - a1^*)^2 + (b2^* - b1^*)^2\}^{1/2} \qquad (3)$$

In Comparative Examples 1 and 2 containing a Tb component, $\Delta b^*$ had a negative value of -3.0 or less, and a pale yellow tint appeared after firing the porcelain, confirming fading. In contrast, the absolute values of $\Delta b^*$ were 3 or less, close to 0, in Examples 1 to 18, and there was almost no fading. It was also confirmed from the small $\Delta E^*$ values of 3.0 or less that little discoloration had occurred.

[Table 1]

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 |
|---|---|---|---|---|---|---|---|---|---|---|---|---|
| Coloring component | Vanadyl oxalate | 0.00 | 0.00 | 0.00 | 0.00 | 0.00100 | 0.0100 | 0.0500 | 0.100 | 0.00 | 0.100 | 0.100 |
| | Vanadyl acetate | 0.00 | 0.00 | 0.00 | 0.00 | 0.00 | 0.00 | 0.00 | 0.00 | 0.100 | 0.00 | 0.00 |
| | Vanadium chloride | 0.0100 | 0.0500 | 0.100 | 0.500 | 0.00 | 0.00 | 0.00 | 0.00 | 0.00 | 0.00 | 0.00 |
| | Nickel acetate hydrate | 0.00 | 0.00 | 0.00 | 0.00 | 0.00 | 0.00 | 0.00 | 0.00 | 0.00 | 0.00 | 0.00 |
| | Chromium chloride hydrate | 0.00 | 0.00 | 0.00 | 0.00 | 0.00 | 0.00 | 0.00 | 0.00 | 0.00 | 0.00 | 0.00 |
| | Terbium chloride hydrate | 0.00 | 0.00 | 0.00 | 0.00 | 0.00 | 0.00 | 0.00 | 0.00 | 0.00 | 0.00 | 0.00 |
| | Terbium acetate hydrate | 0.00 | 0.00 | 0.00 | 0.00 | 0.00 | 0.00 | 0.00 | 0.00 | 0.00 | 0.00 | 0.00 |
| Zr Component | Zirconium chloride oxide hydrate | 0.00 | 0.00 | 0.00 | 0.00 | 0.00 | 0.00 | 0.00 | 0.00 | 0.00 | 0.00 | 0.00 |
| Complexing agent | N,N-Di(2-hydroxyethyl)glycine | 0.00 | 0.00 | 0.00 | 0.00 | 0.00 | 0.00 | 0.00 | 0.00 | 0.00 | 0.00 | 0.00 |
| Solvent | Purified water | 99.99 | 99.95 | 99.90 | 99.50 | 99.999 | 99.99 | 99.95 | 99.90 | 99.90 | 10.0 | 10.0 |
| | Ethanol | 0.00 | 0.00 | 0.00 | 0.00 | 0.00 | 0.00 | 0.00 | 0.00 | 0.00 | 89.90 | 0.00 |
| | Ethylene glycol | 0.00 | 0.00 | 0.00 | 0.00 | 0.00 | 0.00 | 0.00 | 0.00 | 0.00 | 0.00 | 89.90 |
| V ion concentration [mmol/L] | | 0.636 | 3.18 | 6.36 | 31.9 | 0.0645 | 0.645 | 3.23 | 6.46 | 5.41 | 6.46 | 6.46 |
| Zr ion concentration [mol/L] | | 0.00 | 0.00 | 0.00 | 0.00 | 0.00 | 0.00 | 0.00 | 0.00 | 0.00 | 0.00 | 0.00 |

(continued)

| | | Ex. 1 | Ex. 2 | Ex. 3 | Ex. 4 | Ex. 5 | Ex. 6 | Ex. 7 | Ex. 8 | Ex. 9 | Ex. 10 | Ex. 11 |
|---|---|---|---|---|---|---|---|---|---|---|---|---|
| Handling of V component | | Moderate | Moderate | Moderate | Moderate | Good | Good | Good | Good | Good | Good | Good |
| Firing temperature after application of coloring solution [°C]*1 | | 1550 | 1550 | 1550 | 1550 | 1550 | 1550 | 1550 | 1550 | 1550 | 1550 | 1550 |
| Shade after firing (before porcelain build-up) | L1* | 90.65 | 89.62 | 88.84 | 85.37 | 90.48 | 89.19 | 90.36 | 89.73 | 89.98 | 89.31 | 89.49 |
| | a1* | -8.76 | -11.22 | -10.46 | -6.96 | -5.18 | -8.63 | -10.40 | -11.20 | -10.82 | -11.02 | -10.93 |
| | b1* | 24.90 | 36.01 | 42.42 | 53.00 | 13.01 | 22.70 | 32.22 | 40.30 | 36.96 | 40.05 | 39.82 |
| Biaxial flexural strength after firing [MPa] | | 652 | 638 | 645 | 633 | 652 | 644 | 648 | 652 | 641 | 642 | 647 |
| Firing temperature after porcelain build-up [°C] *2 | | 850 | 850 | 850 | 850 | 850 | 850 | 850 | 850 | 850 | 850 | 850 |
| Shade after firing (after porcelain build-up) | L2* | 90.58 | 89.95 | 89.33 | 85.81 | 90.61 | 89.36 | 90.43 | 90.31 | 90.22 | 89.78 | 89.91 |
| | a2* | -9.08 | -11.65 | -10.98 | -7.56 | -4.98 | -8.27 | -10.32 | -11.18 | -10.51 | -10.92 | -10.84 |
| | b2* | 26.51 | 37.85 | 43.87 | 54.01 | 12.85 | 22.55 | 32.01 | 40.23 | 36.78 | 39.91 | 39.57 |
| Shade difference | Δb* | 1.61 | 1.84 | 1.45 | 1.01 | -0.16 | -0.15 | -0.21 | -0.07 | -0.18 | -0.14 | -0.25 |
| | ΔE* | 1.64 | 1.92 | 1.62 | 1.25 | 0.29 | 0.43 | 0.23 | 0.58 | 0.43 | 0.50 | 0.50 |
| *1: In the Table, "Firing temperature" means the highest temperature in firing. Conditions other than firing temperature: Temperature increase from room temperature to 1,550°C at 10°C /min; retention at 1,550°C for 2 hours; temperature decrease at -10°C/min and left to cool from 300°C to room temperature. *2: In the Table, "Firing temperature" means the highest temperature in firing. Conditions other than firing temperature: 5-Minute drying after porcelain build-up and before firing; firing started at 600°C; vacuuming started at 600°C; rate of temperature increase 65°C/min; degree of vacuum 96 kPa; vacuum released at 850°C; 1-minute retention; quenching at room temperature. | | | | | | | | | | | | |

[Table 2]

| Components (parts by mass) | | Ex. 12 | Ex. 13 | Ex. 14 | Ex. 15 | Ex. 16 | Ex. 17 | Ex. 18 | Com. Ex. 1 | Com. Ex. 2 |
|---|---|---|---|---|---|---|---|---|---|---|
| Coloring component | Vanadyl oxalate | 0.0300 | 0.0300 | 0.0300 | 0.0300 | 0.0900 | 0.0500 | 0.0300 | 0.00 | 0.00 |
| | Vanadyl acetate | 0.00 | 0.00 | 0.00 | 0.00 | 0.00 | 0.00 | 0.00 | 0.00 | 0.00 |
| | Vanadium chloride | 0.00 | 0.00 | 0.00 | 0.00 | 0.00 | 0.00 | 0.00 | 0.00 | 0.00 |
| | Nickel acetate hydrate | 1.00 | 1.00 | 1.00 | 1.00 | 0.600 | 0.400 | 1.00 | 0.00 | 0.00 |
| | Chromium chloride hydrate | 0.00 | 0.00 | 0.00 | 0.00 | 0.0300 | 0.250 | 0.00 | 0.00 | 0.00 |
| | Terbium chloride hydrate | 0.00 | 0.00 | 0.00 | 0.00 | 0.00 | 0.00 | 0.00 | 1.00 | 0.00 |
| | Terbium acetate hydrate | 0.00 | 0.00 | 0.00 | 0.00 | 0.00 | 0.00 | 0.00 | 0.00 | 1.00 |
| Zr Component | Zirconium chloride oxide hydrate | 0.00 | 3.00 | 10.0 | 20.0 | 5.00 | 5.00 | 10.0 | 0.00 | 0.00 |
| Complexing agent | N,N-Di(2-hydroxyethyl)glycine | 0.00 | 0.00 | 0.00 | 0.00 | 0.00 | 0.00 | 0.100 | 0.00 | 0.00 |
| Solvent | Purified water | 98.97 | 95.97 | 88.97 | 78.97 | 94.28 | 94.30 | 88.87 | 99.00 | 99.00 |
| | Ethanol | 0.00 | 0.00 | 0.00 | 0.00 | 0.00 | 0.00 | 0.00 | 0.00 | 0.00 |
| | Ethylene glycol | 0.00 | 0.00 | 0.00 | 0.00 | 0.00 | 0.00 | 0.00 | 0.00 | 0.00 |
| V ion concentration [mmol/L] | | 1.96 | 1.99 | 2.07 | 2.20 | 6.02 | 3.34 | 2.07 | 0.00 | 0.00 |
| Zr ion concentration [mol/L] | | 0.00 | 0.0957 | 0.332 | 0.706 | 0.161 | 0.161 | 0.332 | 0.00 | 0.00 |
| | | Ex. 12 | Ex. 13 | Ex. 14 | Ex. 15 | Ex. 16 | Ex. 17 | Ex. 18 | Com. Ex. 1 | Com. Ex. 2 |
| Handling of V component | | Good | Good | Good | Good | Good | Good | Good | | |
| Firing temperature after application of coloring solution [°C] *1 | | 1550 | 1550 | 1550 | 1550 | 1550 | 1550 | 1550 | 1550 | 1550 |
| Shade after firing (before porcelain build-up) | L1* | 72.03 | 72.55 | 71.16 | 70.94 | 73.50 | 66.00 | 71.97 | 85.40 | 84.59 |
| | a1* | 7.41 | 6.02 | 7.88 | 7.45 | 3.71 | 3.20 | 7.98 | -2.86 | -1.01 |
| | b1* | 28.54 | 29.44 | 27.90 | 30.07 | 29.96 | 25.28 | 28.57 | 41.24 | 44.31 |
| Biaxial flexural strength after firing [MPa] | | 644 | 687 | 802 | 784 | 708 | 701 | 793 | 621 | 638 |
| Firing temperature after porcelain build-up [°C] *2 | | 850 | 850 | 850 | 850 | 850 | 850 | 850 | 850 | 850 |
| Shade after firing (after porcelain build-up) | L2* | 72.43 | 72.98 | 71.68 | 71.54 | 74.23 | 66.38 | 72.45 | 86.98 | 86.02 |
| | a2* | 7.21 | 6.00 | 7.67 | 7.23 | 3.97 | 3.42 | 7.72 | -3.37 | -1.32 |
| | b2* | 28.15 | 29.03 | 28.41 | 29.98 | 29.78 | 24.86 | 28.17 | 31.81 | 34.52 |

(continued)

| | | Ex. 12 | Ex. 13 | Ex. 14 | Ex. 15 | Ex. 16 | Ex. 17 | Ex. 18 | Com. Ex. 1 | Com. Ex. 2 |
|---|---|---|---|---|---|---|---|---|---|---|
| Shade difference | $\Delta b^*$ | -0.39 | -0.41 | 0.51 | -0.09 | -0.18 | -0.42 | -0.40 | -9.43 | -9.79 |
| | $\Delta E^*$ | 0.59 | 0.59 | 0.76 | 0.65 | 0.80 | 0.61 | 0.68 | 9.58 | 9.90 |

*1: In the Table, "Firing temperature" means the highest temperature in firing. Conditions other than firing temperature: Temperature increase from room temperature to 1,550°C at 10°C/min; retention at 1,550°C for 2 hours; temperature decrease at -10°C/min and left to cool from 300°C to room temperature.

*2: In the Table, "Firing temperature" means the highest temperature in firing. Conditions other than firing temperature: 5-Minute drying after porcelain build-up and before firing; firing started at 600°C; vacuuming started at 600°C; rate of temperature increase 65°C/min; degree of vacuum 96 kPa; vacuum released at 850°C; 1-minute retention; quenching at room temperature.

INDUSTRIAL APPLICABILITY

With a coloring solution of the present invention, shades with the desired yellow tint can be imparted to a dental ceramic without causing fading, even when the dental ceramic colored with the coloring solution is baked after building up porcelain. This enables a coloring solution of the present invention to be suitably used as a dental ceramic coloring solution. A dental ceramic coloring solution of the present invention is useful given an expected increase in the use of dental ceramic coloring solutions in response to the continuously growing demand for ceramic dental caps and the associated increase in individual demand for better aesthetics.

**Claims**

1.  A coloring solution for coloring a dental ceramic, comprising a coloring component and a solvent, wherein the coloring component comprises a V component.

2.  The coloring solution according to claim 1, wherein the V component is an ion or a complex.

3.  The coloring solution according to claim 1 or 2, wherein the V component has a valency of +IV and/or +V.

4.  The coloring solution according to any one of claims 1 to 3, wherein the V component is an oxidovanadium compound.

5.  The coloring solution according to any one of claims 1 to 4, wherein the V component is a component derived from at least one selected from the group consisting of vanadyl oxalate, vanadyl nitrate, and vanadyl acetate.

6.  The coloring solution according to any one of claims 1 to 5, wherein the content of the V component is 0.0100 to 45.0 mmol/L in terms of a V ion.

7.  The coloring solution according to any one of claims 1 to 6, wherein a dental ceramic after coloring and sintering has an a* of -20 to 20, and a b* of 5 to 60 in (L*,a*,b*) according to L*a*b*color system.

8.  The coloring solution according to any one of claims 1 to 7, wherein L* in (L*,a*,b*) according to L*a*b*color system is 60 to 95.

9.  The coloring solution according to any one of claims 1 to 8, wherein the solvent comprises water and/or an organic solvent.

10. The coloring solution according to claim 9, wherein the organic solvent comprises at least one selected from the group consisting of an alcohol, a glycol, a triol, and a ketone.

11. The coloring solution according to any one of claims 1 to 10, wherein the coloring component comprises a Ni component.

12. The coloring solution according to any one of claims 1 to 11, wherein the coloring component comprises a Cr component.

13. The coloring solution according to any one of claims 1 to 12, wherein the coloring solution further comprises a Zr component.

14. The coloring solution according to any one of claims 1 to 13, wherein the coloring solution does not comprise a Pr component or a Tb component.

15. The coloring solution according to any one of claims 1 to 14, wherein the dental ceramic comprises zirconia as a main component.

16. A dental ceramic supporting a V component on a surface of the dental ceramic.

17. The dental ceramic according to claim 16, wherein the V component is an ion or a complex.

**18.** The dental ceramic according to claim 16 or 17, which comprises zirconia as a main component.

## INTERNATIONAL SEARCH REPORT

| International application No. |
|---|
| **PCT/JP2021/036503** |

**A. CLASSIFICATION OF SUBJECT MATTER**

*A61K 6/78*(2020.01)i; *A61C 13/00*(2006.01)i; *A61C 13/083*(2006.01)i; *A61K 6/818*(2020.01)i
FI:  A61K6/78; A61C13/00 A; A61C13/083; A61K6/818

According to International Patent Classification (IPC) or to both national classification and IPC

**B. FIELDS SEARCHED**

Minimum documentation searched (classification system followed by classification symbols)

A61K6/78; A61C13/00; A61C13/083; A61K6/818

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2010-534245 A (3M INNOVATIVE PROPERTIES COMPANY) 04 November 2010 (2010-11-04) claims, paragraphs [0025], [0028], examples, etc. | 1, 2, 6-12, 14-18 |
| Y | | 3-5, 13 |
| Y | CN 104926370 A (NANCHONG THREE-CIRCLE ELECTRONICS CO., LTD.) 23 September 2015 (2015-09-23) claims, paragraph [0015] | 3-5 |
| Y | CN 111072382 A (SUZHOU DINGAN TECHNOLOGY CO., LTD.) 28 April 2020 (2020-04-28) claims, paragraph [0028] | 3-5, 13 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
**Information on patent family members**

International application No.

**PCT/JP2021/036503**

| Patent document cited in search report | | | Publication date (day/month/year) | Patent family member(s) | Publication date (day/month/year) |
|---|---|---|---|---|---|
| JP | 2010-534245 | A | 04 November 2010 | US 2010/0221683 A1 claims, paragraphs [0035], [0038], examples<br>EP 2025659 A1<br>CN 101778807 A | |
| CN | 104926370 | A | 23 September 2015 | (Family: none) | |
| CN | 111072382 | A | 28 April 2020 | (Family: none) | |

**EP 4 223 270 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007314536 A
- WO 2016068288 A